# EUROPEAN PATENT SPECIFICATION

(11) **EP 3 481 370 B1**
(45) Date of publication and mention of the grant of the patent: **24.03.2021**
(21) Application number: 17733862.1
(22) Date of filing: 03.07.2017
(51) Int. Cl.: A61K 9/00, A61K 47/10, A61K 47/14, A61K 9/10

(54) **PHARMACEUTICAL COMPOSITION COMPRISING BENZYDAMINE**
PHARMAZEUTISCHE ZUSAMMENSETZUNG MIT BENZYDAMIN
COMPOSITION PHARMACEUTIQUE CONTENANT DU BENZYDAMINE

(30) Priority: 08.07.2016 EP 16178742
(43) Date of publication of application: 15.05.2019
(73) Proprietor: Aziende Chimiche Riunite Angelini Francesco A.C.R.A.F. S.p.A., 00181 Roma (IT)
(72) Inventor: FAZIO, Antonello, 04011 APRILIA (Latina) (IT); TONGIANI, Serena, 00046 GROTTAFERRATA (Roma) (IT); DONATI, Luca, 63017 Porto San Giorgio (AP) (IT); MILANESE, Claudio, 00143 ROMA (IT)
(74) Representative: Merli, Silvia
(86) International application number: PCT/EP2017/066438
(87) International publication number: WO 2018/007288

(56) References cited:
- WO-A1-2006/131262
- WO-A1-2016/113194
- CA-A1- 2 009 402
- CN-A- 101 214 218
- CN-A- 101 219 139
- US-A1- 2004 180 965
- US-A1- 2007 031 479

## Description

### FIELD OF THE INVENTION

The present invention relates to a pharmaceutical composition comprising benzydamine. More in particular, the present invention relates to a pharmaceutical cream, cream-gel or gel composition comprising benzydamine having improved tolerability and efficacy for the prevention and treatment of inflammatory and/or infective conditions mainly localized in the genital area, in particular vaginosis, vaginitis and vulvo-vaginitis.

### BACKGROUND OF THE INVENTION

Infections of the female genital apparatus, vaginosis, vaginitis and vulvo-vaginitis, are one of the most widespread gynecological problems, whose incidence seems to be increasing.

Even if the causes are different and numerous, and not always of infectious origin, the majority of diagnoses identifies (i) bacterial vaginosis, i.e. polymicrobial syndromes characterized by a radical modification of the vaginal ecosystem consisting, in particular, in the replacement of the normal lactobacillar flora by pathogens (mainly anaerobes), very often caused or complicated by Gardnerella infections; (ii) Candida vaginitis and vulvo-vaginitis; (iii) Trichomonas vaginalis vaginitis.

Bacterial vaginosis is the most common vaginal and/or vulvo-vaginal infection among women in the fertile age. There are many predisposing factors for these alterations of the bacterial vaginal flora, for example: incorrect hygiene, intrauterine diaphragms, immune deficiencies, diabetes, sexual activity with more partners. The most typical symptom of bacterial vaginosis is abundant, smelly and greyish vaginal loss; itching, vaginal burning and pain during sexual intercourse can rarely be experienced. Pathogenic bacteria, generally Gardnerella vaginalis, which almost completely replace the normal lactobacillar flora, are one of the most common causes of vaginosis.

Vaginitis is an inflammation of the vagina which can affect women of any age; it is called vulvo-vaginitis when it affects also the vulva, i.e. external genitals. In vulvo-vaginitis, fungi of the Candida genus often play a key role as infective agents.

These inflammations can be episodic, recurring (i.e. they appear again after treatment) and in some cases even chronic.

Reference and consolidated therapies (either local or systemic) for the treatment of the above conditions are based on antimycotics, antibacterials, corticosteroids, non steroidal antiinflammatories or xylocaine and derivatives thereof (when itching prevails), or antihistaminics (only orally). In episodic infections there is no need for a change in the therapy, because the advantages outweigh potential risks (side effects, undesired local toxicity reactions, sensitisation); instead, in recurring episodes prevention would undoubtedly be a successful approach.

Several patent applications disclose combinations of active ingredients useful in the treatment of vaginosis, vaginitis and vulvo-vaginitis.

EP2014295 relates to topical compositions containing Zanthoxylum bungeanum extract, 18-beta glycyrrhetic acid, Matricaria chamomilla essential oil, Melaleuca alternifolia essential oil, Curcuma longa or curcumin extract and lactic or propionic acid.

WO2007085020 relates to a pharmaceutical composition for vaginal administration comprising an. estrogen compound, a progesterone compound, and a pharmaceutically acceptable carrier for vaginal administration, wherein the composition is useful in the treatment of urogenital symptoms associated with atrophic vaginitis.

EP0389924 relates to topical dosage form for the treatment of vaginal infections containing purpuromycin.

EP0910377 relates to a pharmaceutical composition for topical administration in the treatment of vaginitis comprising one or more anti-vaginitis medicaments, such as metronidazole and miconazole, and one or more local anaesthetics, such as lidocaine or benzocaine.

EP2034984 relates to a pharmaceutical composition, such as a vaginal suppository or cream, for the treatment of symptoms associated with atrophic vaginitis comprising: a triphenylethylene derivative compound.

WO2016/113194 discloses the formulation of benzydamine hydrochloride in the form of a cream for topical administration comprising benzydamine hydrochloride in an amount of 0.12% w/w, Miglyol® 812 (caprylic/capric triglyceride) in an amount of 3% w/w, Tefose® 63 (PEG-6 and PEG-32 palmitostearate / glycol stearate) in an amount of 18% w/w, and Labrafil® M2130CS (lauroyl macrogol-6-glycerides) in an amount of 3% w/w, together with econazole nitrate in an amount of 1% w/w.

CN 101219139 discloses a suppository comprising benzydamine together with glycerides of fatty acids or polyoxyethylene stearate as excipients.

US 2004/180965 discloses a pharmaceutical composition in a gel form comprising benzydamine and including a polyoxyalkylene block copolimer, such as a poloxamer polymer as a gelling agent and polyethylene glycol.

CA 2009402 discloses a cream composition comprising benzydamine, ethylene glycol and polyethylene glycol palmitostearate, ethoxylated saturated glycerides and saturated neutral glycerides to relieve pain associated with herpes zoster infections, as well as with laser treatment of vulvar condylomata.

The Applicant manufactured and distributed a vaginal cream-gel for the treatment of nonspecific vaginitis containing benzydamine in an amount of 0.5% w/w based on the total weight of the cream-gel.

### SUMMARY OF THE INVENTION

The Applicant faced the problem to provide a pharmaceutical composition comprising benzydamine having improved tolerability and efficacy for the prevention and treatment of inflammatory and/or infective conditions mainly localized in the genital area, in particular vaginosis, vaginitis and vulvo-vaginitis.

The Applicant has found that a pharmaceutical composition comprising (i) at least one ester of glycerin with a C₈-C₁₀ saturated fatty acid, (ii) at least one ester of polyethylene glycol glycerides with a C₁₂-C₁₄ saturated fatty acid, and (iii) at least one ester of polyethylene glycol with a C₁₆-C₁₈ saturated fatty acid, and (iv) benzydamine in an amount equal to or lower than 0.5% w/w with respect to the total weight of the pharmaceutical composition showed an improved tolerability.

The Applicant has also found that a similar improved tolerability is provided by a pharmaceutical composition comprising (i) at least one nonionic water-soluble hydroxyethylcellulose ether having a weight average molecular weight of one million Dalton or more, (ii) at least one polyol, and (iii) benzydamine in an amount lower than 0.5% w/w with respect to the total weight of the pharmaceutical composition, wherein said composition is free of polyethylene glycol ethers of cetearyl and stearyl alcohol, and wherein said composition optionally comprises (iv) at least one ester of glycerin with a C₈-C₁₀ saturated fatty acid, (v) at least one ester of polyethylene glycol glycerides with a C₁₂-C₁₄ saturated fatty acid, and/or (vi) at least one ester of polyethylene glycol with a C₁₆-C₁₈ saturated fatty acid.

Thus, in a first aspect, the present invention relates to a pharmaceutical composition comprising (i) at least one ester of glycerin with a C₈-C₁₀ saturated fatty acid, (ii) at least one ester of polyethylene glycol glycerides with a C₁₂-C₁₄ saturated fatty acid, and (iii) at least one ester of polyethylene glycol with a C₁₆-C₁₈ saturated fatty acid, and (iv) benzydamine in an amount equal to or lower than 0.5% w/w with respect to the total weight of the pharmaceutical composition, provided that, when benzydamine is present as benzydamine hydrochloride in an amount of 0.12% w/w, together with Miglyol® 812 in an amount of 3% w/w, Tefose® 63 in an amount of 18% w/w, and Labrafil® M2130CS in an amount of 3% w/w, the composition does not comprise econazole nitrate.

In a second aspect, the present invention relates to a pharmaceutical composition comprising (i) at least one nonionic water-soluble hydroxyethylcellulose ether having a weight average molecular weight of one million Dalton or more, (ii) at least one polyol, and (iii) benzydamine in an amount lower than 0.5% w/w with respect to the total weight of the pharmaceutical composition, wherein said composition is free of polyethylene glycol ethers of cetearyl and stearyl alcohol, and wherein said composition optionally comprises (iv) at least one ester of glycerin with a C₈-C₁₀ saturated fatty acid, (v) at least one ester of polyethylene glycol glycerides with a C₁₂-C₁₄ saturated fatty acid, and/or (vi) at least one ester of polyethylene glycol with a C₁₆-C₁₈ saturated fatty acid The scope of the invention is defined by the claims. Any references in the description to methods of treatment refer to the products (compounds, pharmaceutical compositions and medicaments) of the present invention for use in a method for treatment of the human or animal body by therapy or diagnosis. Any "embodiment" or "example" which is disclosed in the description but is not covered by the claims should be considered as presented for illustrative purpose only.

### DETAILED DESCRIPTION OF THE INVENTION

According to a first aspect, the present invention relates to a pharmaceutical composition comprising (i) at least one ester of glycerin with a C₈-C₁₀ saturated fatty acid, (ii) at least one ester of polyethylene glycol glycerides with a C₁₂-C₁₄ saturated fatty acid, and (iii) at least one ester of polyethylene glycol with a C₁₆-C₁₈ saturated fatty acid, and (iv) benzydamine in an amount equal to or lower than 0.5% w/w with respect to the total weight of the pharmaceutical composition, provided that, when benzydamine is present as benzydamine hydrochloride in an amount of 0.12% w/w, together with Miglyol® 812 in an amount of 3% w/w, Tefose® 63 in an amount of 18% w/w, and Labrafil® M2130CS in an amount of 3% w/w, the composition does not comprise econazole nitrate.

The pharmaceutical composition according to the present invention preferably comprises at least one ester of glycerin with a C₈-C₁₀ saturated fatty acid in an amount lower than 10% w/w with respect to the total weight of the pharmaceutical composition.

More preferably, the pharmaceutical composition according to the present invention comprises at least one ester of glycerin with a C₈-C₁₀ saturated fatty acid in an amount lower than 7% w/w with respect to the total weight of the pharmaceutical composition.

Even more preferably, the pharmaceutical composition according to the present invention comprises at least one ester of glycerin with a C₈-C₁₀ saturated fatty acid in an amount lower than 5% w/w with respect to the total weight of the pharmaceutical composition.

Preferably, the pharmaceutical composition according to the present invention comprises at least one ester of glycerin with a C₈-C₁₀ saturated fatty acid in an amount higher than 0.5% w/w with respect to the total weight of the pharmaceutical composition.

More preferably, the pharmaceutical composition according to the present invention comprises at least one ester of glycerin with a C₈-C₁₀ saturated fatty acid in an amount higher than 1% w/w with respect to the total weight of the pharmaceutical composition.

Even more preferably, the pharmaceutical composition according to the present invention comprises at least one ester of glycerin with a C₈-C₁₀ saturated fatty acid in an amount higher than 2% w/w with respect to the total weight of the pharmaceutical composition.

According to a preferred embodiment of the present invention, the pharmaceutical composition comprises at least one ester of glycerin with a C₈-C₁₀ saturated fatty acid in an amount ranging from 1.5% to 6% w/w with respect to the total weight of the pharmaceutical composition.

According to a more preferred embodiment of the present invention, the pharmaceutical composition comprises at least one ester of glycerin with a C₈-C₁₀ saturated fatty acid in an amount ranging from 2.5% to 4% w/w with respect to the total weight of the pharmaceutical composition.

Useful esters of glycerin with a C₈-C₁₀ saturated fatty acid are mono-, di-, or tri-glycerides of capric acid and/or caprylic acid, and mixture thereof.

Esters of glycerin with a C₈-C₁₀ saturated fatty acid are commercially available from several suppliers under different commercial names, such as for example Captex® 300 and Captex® 355 (Abitec Corporation), Myritol® 312 and Myritol® 318 (BASF), Miglyol® 810 and Miglyol® 812 (Sasol), Tegosoft® CT (Evonik), Crodamol™ GTCC (Croda), BergaBest™ MCT-Oil (Berg & Schmidt), and Dermarol™ CCT (CISME Italy).

The pharmaceutical composition according to the present invention preferably comprises at least one ester of polyethylene glycol glycerides with a C₁₂-C₁₄ saturated fatty acid in an amount lower than 10% w/w with respect to the total weight of the pharmaceutical composition.

More preferably, the pharmaceutical composition according to the present invention comprises at least one ester of polyethylene glycol glycerides with a C₁₂-C₁₄ saturated fatty acid in an amount lower than 7% w/w with respect to the total weight of the pharmaceutical composition.

Even more preferably, the pharmaceutical composition according to the present invention comprises at least one ester of polyethylene glycol glycerides with a C₁₂-C₁₄ saturated fatty acid in an amount lower than 5% w/w with respect to the total weight of the pharmaceutical composition.

Preferably, the pharmaceutical composition according to the present invention comprises at least one ester of polyethylene glycol glycerides with a C₁₂-C₁₄ saturated fatty acid in an amount higher than 0.5% w/w with respect to the total weight of the pharmaceutical composition.

More preferably, the pharmaceutical composition according to the present invention comprises at least one ester of polyethylene glycol glycerides with a C₁₂-C₁₄ saturated fatty acid in an amount higher than 1% w/w with respect to the total weight of the pharmaceutical composition.

Even more preferably, the pharmaceutical composition according to the present invention comprises at least one ester of polyethylene glycol glycerides with a C₁₂-C₁₄ saturated fatty acid in an amount higher than 2% w/w with respect to the total weight of the pharmaceutical composition.

According to a preferred embodiment of the present invention, the pharmaceutical composition comprises at least one ester of polyethylene glycol glycerides with a C₁₂-C₁₄ saturated fatty acid in an amount ranging from 1.5% to 6% w/w with respect to the total weight of the pharmaceutical composition.

According to a more preferred embodiment of the present invention, the pharmaceutical composition comprises at least one ester of polyethylene glycol glycerides with a C₁₂-C₁₄ saturated fatty acid in an amount ranging from 2.5% to 4% w/w with respect to the total weight of the pharmaceutical composition.

Useful esters of polyethylene glycol glycerides with a C₁₂-C₁₄ saturated fatty acid are polyethylene glycol mono- or di-glycerides with lauric acid and/or myristic acid, and mixture thereof. The polyethylene glycol chain linked to the glyceride can have from 4 to 10 oxyethylene monomeric units, preferably from 6 to 8 monomeric oxyethylene units.

Esters of polyethylene glycol glycerides with a C₁₂-C₁₄ saturated fatty acid are commercially available from several suppliers under different commercial names, such as for example Acconon® C-44 (Abitec Corporation), Labrafil® M2130CS and Gelucire® 44/14 (Gattefosse).

The pharmaceutical composition according to the present invention preferably comprises at least one ester of polyethylene glycol with a C₁₆-C₁₈ saturated fatty acid in an amount lower than 30% w/w with respect to the total weight of the pharmaceutical composition.

More preferably, the pharmaceutical composition according to the present invention comprises at least one ester of polyethylene glycol with a C₁₆-C₁₈ saturated fatty acid in an amount lower than 25% w/w with respect to the total weight of the pharmaceutical composition.

Preferably, the pharmaceutical composition according to the present invention comprises at least one ester of polyethylene glycol with a C₁₆-C₁₈ saturated fatty acid in an amount higher than 1% w/w with respect to the total weight of the pharmaceutical composition.

More preferably, the pharmaceutical composition according to the present invention comprises at least one ester of polyethylene glycol with a C₁₆-C₁₈ saturated fatty acid in an amount higher than 2% w/w with respect to the total weight of the pharmaceutical composition.

According to a preferred embodiment of the present invention, the pharmaceutical composition comprises at least one ester of polyethylene glycol with a C₁₆-C₁₈ saturated fatty acid in an amount ranging from 5% to 20% w/w with respect to the total weight of the pharmaceutical composition.

Useful esters of polyethylene glycol with a C₁₆-C₁₈ saturated fatty acid are esters of polyethylene glycols having from 6 to 32 oxyethylene monomeric units with palmitic acid and/or stearic acid, and mixture thereof.

Esters of polyethylene glycol with a C₁₆-C₁₈ saturated fatty acid are commercially available from several suppliers under different commercial names, such as for example Mirj™ S8, S25, S40 and S100 (Croda), Gelucire® 48/16, Tefose® 1500 CG, and Tefose 63 (Gattefosse), Simulsol™ M45PHA and M52PHA (Seppic).

Preferably, the pharmaceutical composition of the present invention does not comprise econazole nitrate when benzydamine is present together with Miglyol® 812, Tefose® 63 and Labrafil® M2130CS. More preferably, the pharmaceutical composition of the present invention does not comprise econazole nitrate.

In a second aspect, the present invention relates to a pharmaceutical composition comprising (i) at least one nonionic water-soluble hydroxyethylcellulose ether having a weight average molecular weight of one million Dalton or more, (ii) at least one polyol, and (iii) benzydamine in an amount lower than 0.5% w/w with respect to the total weight of the pharmaceutical composition, wherein said composition is free of polyethylene glycol ethers of cetearyl and stearyl alcohol.

Useful nonionic water-soluble hydroxyethylcellulose ethers (i) have an average degree of substitution between 1 and 2 and an average degree of substituents between 2 and 3. The degree of substitution is the average number of substituted hydroxyl in the cellulose chain. The degree of substituents is the average number of ethylene oxide units linked to each glucose unit of the cellulose chain. The nonionic water-soluble hydroxyethylcellulose ethers preferably have a weight average molecular weight of from 1.0 to 1.5 x 10⁶ Dalton.

Nonionic water-soluble hydroxyethylcellulose ethers are commercially available from several suppliers under different commercial names, such as for example, Natrosol®250HHR, Natrosol®250H4R, and Natrosol®250HR (Hercules Inc.).

The pharmaceutical composition according to the present invention preferably comprises at least one nonionic water-soluble hydroxyethylcellulose ether in an amount lower than 3% w/w with respect to the total weight of the pharmaceutical composition.

More preferably, the pharmaceutical composition according to the present invention comprises at least one nonionic water-soluble hydroxyethylcellulose ether in an amount lower than 2.5% w/w with respect to the total weight of the pharmaceutical composition.

Even more preferably, the pharmaceutical composition according to the present invention comprises at least one nonionic water-soluble hydroxyethylcellulose ether in an amount lower than 2% w/w with respect to the total weight of the pharmaceutical composition.

Preferably, the pharmaceutical composition according to the present invention comprises at least one nonionic water-soluble hydroxyethylcellulose ether in an amount higher than 0.5% w/w with respect to the total weight of the pharmaceutical composition.

More preferably, the pharmaceutical composition according to the present invention comprises at least one nonionic water-soluble hydroxyethylcellulose ether in an amount higher than 1% w/w with respect to the total weight of the pharmaceutical composition.

Even more preferably, the pharmaceutical composition according to the present invention comprises at least one nonionic water-soluble hydroxyethylcellulose ether in an amount higher than 1.5% w/w with respect to the total weight of the pharmaceutical composition.

According to a preferred embodiment of the present invention, the pharmaceutical composition comprises at least one nonionic water-soluble hydroxyethylcellulose ether in an amount ranging from 0.8% to 2.7% w/w with respect to the total weight of the pharmaceutical composition.

According to a more preferred embodiment of the present invention, the pharmaceutical composition comprises at least one nonionic water-soluble hydroxyethylcellulose ether in an amount ranging from 1.3% to 2.3% w/w with respect to the total weight of the pharmaceutical composition.

Useful polyols (ii) are polyalcohols having two or more hydroxy groups linked to a carbon chain having from 3 to 6 carbon atoms, such as, for example, glycerol, erythritol, arabitol, xylitol, ribitol, mannitol, sorbitol, galactitol, and inositol. Glycerol is a particularly preferred polyol useful in the present invention.

The pharmaceutical composition according to the present invention preferably comprises at least one polyol in an amount lower than 20% w/w with respect to the total weight of the pharmaceutical composition.

More preferably, the pharmaceutical composition according to the present invention comprises at least one polyol in an amount lower than 18% w/w with respect to the total weight of the pharmaceutical composition.

Even more preferably, the pharmaceutical composition according to the present invention comprises at least one polyol in an amount lower than 15% w/w with respect to the total weight of the pharmaceutical composition.

Preferably, the pharmaceutical composition according to the present invention comprises at least one polyol in an amount higher than 1% w/w with respect to the total weight of the pharmaceutical composition.

More preferably, the pharmaceutical composition according to the present invention comprises at least one polyol in an amount higher than 3% w/w with respect to the total weight of the pharmaceutical composition.

Even more preferably, the pharmaceutical composition according to the present invention comprises at least one polyol in an amount higher than 5% w/w with respect to the total weight of the pharmaceutical composition.

According to a preferred embodiment of the present invention, the pharmaceutical composition comprises at least one polyol in an amount ranging from 6% to 14% w/w with respect to the total weight of the pharmaceutical composition.

According to a more preferred embodiment of the present invention, the pharmaceutical composition comprises at least one polyol in an amount ranging from 8% to 12% w/w with respect to the total weight of the pharmaceutical composition.

Optionally, the composition according to the second aspect of the present invention can additionally comprise (iv) at least one ester of glycerin with a C₈-C₁₀ saturated fatty acid, (v) at least one ester of polyethylene glycol glycerides with a C₁₂-C₁₄ saturated fatty acid, and/or (vi) at least one ester of polyethylene glycol with a C₁₆-C₁₈ saturated fatty acid in the same amounts described above.

Useful examples of esters of glycerin with a C₈-C₁₀ saturated fatty acid are the same described above such as Captex® 300 and Captex® 355 (Abitec Corporation), Myritol® 312 and Myritol® 318 (BASF), Miglyol® 810 and Miglyol® 812 (Sasol), Tegosoft® CT (Evonik), Crodamol™ GTCC (Croda), BergaBest™ MCT-Oil (Berg & Schmidt), and Dermarol™ CCT (CISME Italy).

Useful examples of ester of polyethylene glycol with a C₁₆-C₁₈ saturated fatty acid are the same described above, such as Mirj™ S8, S25, S40 and S100 (Croda), Gelucire® 48/16, Tefose® 1500 CG, and Tefose 63 (Gattefosse), Simulsol™ M45PHA and M52PHA (Seppic).

Preferably, the pharmaceutical composition according to the present invention comprises benzydamine in an amount lower than 0.5% w/w with respect to the total weight of the pharmaceutical composition.

More preferably, the pharmaceutical composition according to the present invention comprises benzydamine in an amount lower than 0.4% w/w with respect to the total weight of the pharmaceutical composition.

Even more preferably, the pharmaceutical composition according to the present invention comprises benzydamine in an amount lower than 0.3% w/w with respect to the total weight of the pharmaceutical composition.

Preferably, the pharmaceutical composition according to the present invention comprises benzydamine in an amount higher than 0.01% w/w with respect to the total weight of the pharmaceutical composition.

More preferably, the pharmaceutical composition according to the present invention comprises benzydamine in an amount higher than 0.05% w/w with respect to the total weight of the pharmaceutical composition.

Even more preferably, the pharmaceutical composition according to the present invention comprises benzydamine in an amount higher than 0.1% w/w with respect to the total weight of the pharmaceutical composition.

According to a preferred embodiment of the present invention, the pharmaceutical composition comprises benzydamine in an amount ranging from 0.03% to 0.3% w/w with respect to the total weight of the pharmaceutical composition.

According to a more preferred embodiment of the present invention, the pharmaceutical composition comprises benzydamine in an amount ranging from 0.1% to 0.25 w/w with respect to the total weight of the pharmaceutical composition.

The pharmaceutical composition according to the present invention preferably comprises an aqueous dispersion of (i) at least one ester of glycerin with a C₈-C₁₀ saturated fatty acid, (ii) at least one ester of polyethylene glycol glycerides with a C₁₂-C₁₄ saturated fatty acid, (iii) at least one ester of polyethylene glycol with a C₁₆-C₁₈ saturated fatty acid, and (iv) benzydamine in an amount equal to or lower than 0.5% w/w with respect to the total weight of the pharmaceutical composition.

The pharmaceutical composition according to the present invention preferably comprises an aqueous dispersion of (i) at least one nonionic water-soluble hydroxyethylcellulose ether having a weight average molecular weight of one million Dalton or more, (ii) at least one polyol, and (iii) benzydamine in an amount lower than 0.5% w/w with respect to the total weight of the pharmaceutical composition, wherein said composition is free of polyethylene glycol ethers of cetearyl and stearyl alcohol, and wherein said composition optionally comprises (iv) at least one ester of glycerin with a C₈-C₁₀ saturated fatty acid, (v) at least one ester of polyethylene glycol glycerides with a C₁₂-C₁₄ saturated fatty acid, and/or (vi) at least one ester of polyethylene glycol with a C₁₆-C₁₈ saturated fatty acid.

The pharmaceutical composition of the present invention in the form of aqueous dispersion comprises water in an amount higher than 40% w/w, preferably higher than 50% w/w, and more preferably higher than 60% w/w with respect to the total weight of the composition. Most preferably, water is comprised in an amount higher than 70% w/w with respect to the total weight of the pharmaceutical composition.

Preferably, the pharmaceutical composition according to the present invention is for topical use.

Preferably, the pharmaceutical composition according to the present invention is prepared in suitable dosage forms, such as creams, ointments, lotions, cream-gels, gels, foams, vaginal douches, or solutions for external use.

More preferably, said dosage form is cream, cream-gel, gel, vaginal douche or solution for external use.

Even more preferably, said dosage form is cream, cream-gel, or gel.

Most preferably, the pharmaceutical composition according to the first aspect of the present invention is in the form of a cream, and the pharmaceutical composition according to the second aspect of the present invention is in the form of a gel or a cream-gel.

The pharmaceutical composition according to the present invention may comprises other pharmaceutically acceptable excipients.

Useful pharmaceutically acceptable excipients can be selected from the group comprising emollients, thickeners, preservative, stabilizers, buffers, and the like.

Advantageously, the pharmaceutical composition comprises at least one preservative, such as, for example, benzoic acid, ascorbic acid, and/or propyl gallate.

Advantageously, the pharmaceutical composition comprises at least one stabilizer, such as, for example, ethylenediaminetetraacetic acid (EDTA), N-(hydroxyethyl)-ethylenediaminetriacetic acid (HEDTA), nitrilotriacetic acid (NTA), and/or diethylenetriaminepentaacetic acid (DTPA).

The examples that follow are intended for further illustration of the present invention, though without limiting it.

### EXAMPLES

### Tolerability test 1

An in vitro test on vaginal mucosa cells was conducted by using cream-gels and aqueous solutions of benzydamine at different concentrations. A placebo cream-gel free of benzydamine hydrochloride was also tested. The base cream-gel had composition reported in the following table 1. The samples under investigation had the benzydamine hydrochloride concentration as reposted in the following table 2.

**TABLE 1**

| INGREDIENT | % W/W |
|---|---|
| Sodium Citrate | 0.45 |
| Citric Acid | 0.07 |
| Ceteareth 20 | 2.0 |
| Neutral oil | 2.5 |
| HEC | 1.8 |
| Propylenic glycol | 8.0 |
| Benzoic acid | 0.2 |
| Water q.s. | 100 |

Ceteareth-20 is the polyethylene glycol ether of cetearyl and stearyl alcohol, where 20 indicates the average number of ethylene oxide residues in the polyoxyethylene chain.

HEC is a nonionic water-soluble hydroxyethylcellulose ether having a weight average molecular weight of about 1.3 x 10⁶ Dalton.

Neutral oil is a mixture of esters of glycerin with a caprylic and capric fatty acid.

**TABLE 2**

| SAMPLE | COMPOSITION | % W/W |
|---|---|---|
| 1 | Aqueous solution | 0.06 |
| 2 | Aqueous solution | 0.12 |
| 3 | Aqueous solution | 0.25 |
| 4 | Aqueous solution | 0.50 |
| 5 | Cream-gel | 0.12 |
| 6 | Cream-gel | 0.25 |
| 7 | Cream-gel | 0.50 |
| PL | Cream-gel | - |

The cell viability was measured after 1 hour and after 24 hour from application of the sample to the vaginal mucosa and the results were normalized to an untreated sample of vaginal mucosa (CONTROL).

The results are reported in the following table 3.

**TABLE 3**

| SAMPLE | VIABILITY AFTER 1 HOUR | VIABILITY AFTER 24 HOUR |
|---|---|---|
| CONTROL | 100.00 | 100.00 |
| 1 | 106.59 | 101.61 |
| 2 | 106.20 | 93.03 |
| 3 | 110.87 | 78.23 |
| 4 | 60.46 | 21.24 |
| 5 | 0.84 | 0.53 |
| 6 | 1.16 | 0.59 |
| 7 | 0.78 | 0.65 |
| PL | 0.81 | 0.60 |

The data clearly showed that benzydamine aqueous solution up to 0.25% w/w are very well tolerated by vaginal mucosa, while a concentration of 0.50% w/w started to show a lower tolerability.

On the other hand, the cream-gel formulations of benzydamine were not tolerated at any concentration, but this was due to the composition of the cream-gel rather than to the benzydamine, as demonstrated by the placebo sample.

### Tolerability test 2

Different formulations were prepared and tested for their tolerability as illustrated in the tolerability test 1.

The compositions of the base formulations are illustrated in the following table 4. All amounts are expressed in percent by weight based on the weight of the total composition.

**TABLE 4**

| Composition | 21 | 22 | 23 | 24 | 25 | 26 | 27 | 28 |
|---|---|---|---|---|---|---|---|---|
| Benzoic acid | 0.2 | 0.2 | 0.2 | 0.2 | 0.2 | 0.2 | 0.2 | 0.2 |
| Sodium Citrate | 0.45 | 0.45 | 0.45 | 0.45 | 0.45 | 0.45 | 0.45 | 0.45 |
| Citric Acid | 0.07 | 0.07 | 0.07 | 0.07 | 0.07 | 0.07 | 0.07 | 0.07 |
| HEC | 1.8 | 1.8 | 1.8 | 1.8 | 1.8 | 1.8 | 1.8 | 1.8 |
| Ceteareth 20 | 2.0 | 1.0 | 1.0 | 2.0 | -- | 2.0 | -- | -- |
| Neutral oil | 2.5 | 1.25 | 2.5 | 2.5 | -- | 2.5 | 2.5 | 1.25 |
| Emulsifier | -- | -- | -- | -- | -- | -- | 5.0 | 2.5 |
| Surfactant | -- | -- | -- | -- | -- | -- | 2.5 | 1.25 |
| Propylenic glycol | -- | -- | -- | -- | -- | 8 | -- | -- |
| Glycerol | 10 | 10 | 10 | -- | 10 | -- | 10 | 10 |
| Water q.s. | 100 | 100 | 100 | 100 | 100 | 100 | 100 | 100 |

Ceteareth-20 is the polyethylene glycol ether of cetearyl and stearyl alcohol, where 20 indicates the average number of ethylene oxide residues in the polyoxyethylene chain.

HEC is a nonionic water-soluble hydroxyethylcellulose ether having a weight average molecular weight of about one million Dalton.

Neutral oil is a mixture of esters of glycerin with a caprylic and capric fatty acid.

Emulsifier is a mixture of PEG-6 stearate and PEG-32 stearate.

Surfactant is a mixture of lauroyl macrogol-6 glycerides.

The test results, normalized to an untreated sample of vaginal mucosa (CONTROL), are reported in the following table 5.

**TABLE 5**

| SAMPLE | VIABILITY AFTER 1 HOUR | VIABILITY AFTER 24 HOUR |
|---|---|---|
| CONTROL | 100.00 | 100.00 |
| 21 | 0.78 | 0.74 |
| 22 | 3.82 | 0.75 |
| 23 | 3.19 | 0.69 |
| 24 | 0.83 | 0.72 |
| 25 | 86.75 | 84.28 |
| 26 | 1.08 | 0.75 |
| 27 | 98.40 | 90.43 |
| 28 | 94.96 | 95.49 |

The results surprisingly showed that the removal of Ceteareth-20 strongly improved the tolerability of the formulation 25 (having the consistency of a gel) on the mucosa vaginal cells, which was further even improved by the use of the mixture of emulsifier and surfactant of samples 27 and 28 (having the consistency of a cream-gel).

### Tolerability test 3

Formulation 25 and a formulation 54 having a composition similar to that of formulation 27, containing different concentrations of benzydamine hydrochloride, were prepared and tested for their tolerability as illustrated in the tolerability test 1.

The compositions of the formulations are illustrated in the following table 6. All amounts are expressed in percent by weight based on the weight of the total composition.

**TABLE 6**

| | Gel formulations | | | | | Cream formulations | | | | |
|---|---|---|---|---|---|---|---|---|---|---|
| | 25 | 50 | 51 | 52 | 53 | 54 | 55 | 56 | 57 | 58 |
| Glycerol | 10 | 10 | 10 | 10 | 10 | -- | -- | -- | -- | -- |
| Benzoic acid | 0.2 | 0.2 | 0.2 | 0.2 | 0.2 | 0.2 | 0.2 | 0.2 | 0.2 | 0.2 |
| Sodium Citrate | 0.45 | 0.45 | 0.45 | 0.45 | 0.45 | 0.40 | 0.40 | 0.40 | 0.40 | 0.40 |
| Citric Acid | 0.07 | 0.07 | 0.07 | 0.07 | 0.07 | 0.2 | 0.2 | 0.2 | 0.2 | 0.2 |
| HEC | 1.8 | 1.8 | 1.8 | 1.8 | 1.8 | -- | -- | -- | -- | -- |
| Neutral oil | -- | -- | -- | -- | -- | 3.0 | 3.0 | 3.0 | 3.0 | 3.0 |
| Emulsifier | -- | -- | -- | -- | -- | 18.0 | 18.0 | 18.0 | 18.0 | 18.0 |
| Surfactant | -- | -- | -- | -- | -- | 3.0 | 3.0 | 3.0 | 3.0 | 3.0 |
| Benzydamine hydrochloride | -- | 0.50 | 0.25 | 0.12 | 0.06 | -- | 0.50 | 0.35 | 0.25 | 0.12 |

The test results, normalized to an untreated sample of vaginal mucosa (CONTROL), are reported in the following table 7.

**TABLE 7**

| SAMPLE | VIABILITY AFTER 24 HOUR |
|---|---|
| CONTROL | 100.00 |
| 25 | 79.20 |
| 50 | 0.80 |
| 51 | 22.90 |
| 52 | 81.70 |
| 53 | 92.30 |
| 54 | 42.40 |
| 55 | 55.63 |
| 56 | 59.90 |
| 57 | 50.30 |
| 58 | 69.20 |

The results clearly showed that the formulations in the form of cream were tolerated at any tested concentration of benzydamine, with optimal results for sample 58 having a concentration of benzydamine hydrochloride of 0.12 % w/w.

Further, the results also showed that the formulations in the form of gels, having a higher release speed, were tolerated up to a concentration of benzydamine hydrochloride of 0.12 % w/w.

### Stability test

Different cream formulations based on formulation 58 were tested for stability by storing them at temperature of 40°C for two months. After storage, the emulsion was visually inspected for detecting any phase separation or granule formation, chemically analyzed for detecting the presence of benzydamine N-oxide, and tested for their tolerability as illustrated in the tolerability test 1.

The composition of the tested formulations is illustrated in the following tables 8A and 8B. All amounts are expressed in percent by weight based on the weight of the total composition.

**TABLE 8A**

| Composition | 60 | 61 | 62 | 63 | 64 | 65 | 66 | 67 |
|---|---|---|---|---|---|---|---|---|
| Benzydamine hydrochloride | 0.12 | 0.12 | 0.12 | 0.12 | 0.12 | 0.12 | 0.12 | 0.12 |
| Neutral oil | 3.0 | 3.0 | 3.0 | 3.0 | 3.0 | 3.0 | 3.0 | 3.0 |
| Emulsifier | 18.0 | 18.0 | 18.0 | 18.0 | 18.0 | 18.0 | 18.0 | 18.0 |
| Surfactant | 3.0 | 3.0 | 3.0 | 3.0 | 3.0 | 3.0 | 3.0 | 3.0 |
| Benzoic acid | 0.2 | 0.2 | 0.2 | 0.2 | 0.2 | 0.2 | 0.2 | 0.2 |
| Sodium Citrate | 0.40 | 0.40 | 0.40 | 0.40 | 0.40 | 0.40 | 0.40 | 0.40 |
| Citric Acid | 0.2 | 0.2 | 0.2 | 0.2 | 0.2 | 0.2 | 0.2 | 0.2 |
| BHA | 0.05 | - | - | - | 0.01 | - | 0.01 | |
| Pyruvic acid | - | 0.085 | 0.128 | - | - | - | - | - |
| Vitamin E | - | - | - | 0.05 | 0.05 | 0.05 | 0.05 | |
| Ascorbyl palmitate | - | - | - | - | - | 0.05 | 0.05 | 0.05 |
| Propylgallate | - | - | - | - | - | - | - | - |
| EDTA | - | - | - | - | - | - | - | - |
| Water q.s. | 100 | 100 | 100 | 100 | 100 | 100 | 100 | 100 |

**TABLE 8B**

| Composition | 68 | 69 | 70 | 71 | 72 | 73 | 74 |
|---|---|---|---|---|---|---|---|
| Benzydamine hydrochloride | 0.12 | 0.12 | 0.12 | 0.12 | 0.12 | 0.12 | 0.12 |
| Neutral oil | 3.0 | 3.0 | 3.0 | 3.0 | 3.0 | 3.0 | 3.0 |
| Emulsifier | 18.0 | 18.0 | 18.0 | 18.0 | 18.0 | 18.0 | 18.0 |
| Surfactant | 3.0 | 3.0 | 3.0 | 3.0 | 3.0 | 3.0 | 3.0 |
| Benzoic acid | 0.2 | 0.2 | 0.2 | 0.2 | 0.2 | 0.2 | 0.2 |
| Sodium Citrate | 0.4 | 0.4 | - | - | - | - | - |
| Citric Acid | 0.2 | 0.2 | - | - | - | - | - |
| BHA | - | - | - | - | - | - | - |
| Pyruvic acid | - | - | - | - | - | - | - |
| Vitamin E | - | - | - | - | - | - | - |
| Ascorbyl palmitate | - | - | - | - | - | - | - |
| Propylgallate | 0.05 | 0.1 | 0.05 | 0.1 | 0.1 | 0.1 | 0.1 |
| EDTA | - | - | - | - | 0.025 | 0.05 | 0.1 |
| Water q.s. | 100 | 100 | 100 | 100 | 100 | 100 | 100 |

The test results are reported in the following table 9. The stability and tolerability were evaluated by using the following score:
1 Bad
2 Sufficient
3 Good
4 Very good

**TABLE 9**

| SAMPLE | EMULSION STABILITY | CHEMICAL STABILITY | TOLERABILITY |
|---|---|---|---|
| 60 | 3 | 3 | 1 |
| 61 | 3 | 3 | 1 |
| 62 | 3 | 3 | 1 |
| 63 | 3 | 1 | - |
| 64 | 3 | 1 | - |
| 65 | 3 | 1 | - |
| 66 | 3 | 1 | - |
| 67 | 3 | 1 | - |
| 68 | 3 | 2 | 2 |
| 69 | 3 | 2 | 2 |
| 70 | 2 | 3 | 2 |
| 71 | 2 | 3 | 3 |
| 72 | 3 | 3 | 4 |
| 73 | 4 | 3 | 4 |
| 74 | 4 | 4 | 4 |

Formulations 60 to 62 containing BHA or pyruvic acid were sufficiently stable, but showed a very bad tolerability.

Formulations 63 to 67 containing Vitamin E and/or ascorbyl palmitate, also in combination with BHA were found not chemically stable, with formation of benzydamine N-oxide (the test of tolerability was not even made for these compositions).

Formulations 68 and 69 were stable, but with formation of black dots and a yellowish color after storage. The removal of the buffer system (Sodium Citrate / Citric Acid), as in formulations 70 and 71, improved this defect, but worsened the emulsion stability.

Formulations 72 to 74, wherein the buffer system has been removed and a stabilizer EDTA was added, surprisingly provided the best results.

### In vivo preclinical test

Female New Zealand White rabbits were treated once a day by intravaginal route with 1 ml of Placebo Vaginal Cream or Benzydamine Hydrochloride Vaginal Cream (6 animals/group of treatment + 2 for recovery) for 4 weeks with an interim sacrifice at 2 weeks.

The composition of Placebo Vaginal Cream or Benzydamine Hydrochloride Vaginal Cream are illustrated in the following table 10. All amounts are expressed in percent by weight based on the weight of the total composition.

**TABLE 10**

| Composition | Placebo Vaginal Cream | Benzydamine Hydrochloride Vaginal Cream |
|---|---|---|
| Benzydamine hydrochloride | - | 0.12 |
| Neutral oil | 3.0 | 3.0 |
| Emulsifier | 18.0 | 18.0 |
| Surfactant | 3.0 | 3.0 |
| Benzoic acid | 0.2 | 0.2 |
| Propylgallate | 0.1 | 0.1 |
| EDTA | 0.1 | 0.1 |
| Water q.s. | 100 | 100 |

At the end of the treatment period, all animals were sacrificed except recovery animals, which were kept for a 2-week treatment-free period.

The results of the test are summarized hereinbelow.

Once daily intravaginal administration of BHV Cream, to female New Zealand White rabbits, for 2 or 4 weeks, resulted in very limited systemic exposure. There were no mortalities, adverse clinical signs or local reactions, and no macroscopic changes among examined parameters were considered to be related to BHV Cream.

Histologically, ulcerative changes along with inflammation, cellular debris/inflammatory cells in the lumen and decreased amount of mucus were observed in both groups of animals treated with the placebo or the BHV cream. Following the 2-week treatment-free period, only minor changes were seen in the vagina indicating a clear recovery process.

In conclusion, the BHV Cream is considered a well tolerated product for the treatment of inflammatory and/or infective conditions mainly localized in the genital area, in particular vaginosis, vaginitis and vulvo-vaginitis.

## Claims

1. A pharmaceutical composition comprising (i) at least one ester of glycerin with a C₈-C₁₀ saturated fatty acid, (ii) at least one ester of polyethylene glycol glycerides with a C₁₂-C₁₄ saturated fatty acid, and (iii) at least one ester of polyethylene glycol with a C₁₆-C₁₈ saturated fatty acid, and (iv) benzydamine in an amount equal to or lower than 0.5% w/w with respect to the total weight of the pharmaceutical composition,
provided that, when benzydamine is present as benzydamine hydrochloride in an amount of 0.12% w/w, together with caprylic/ capric triglyceride (Miglyol® 812) in an amount of 3% w/w, PEG-6 and PEG-32 palmitostearate / glycol stearate (Tefose® 63) in an amount of 18% w/w, and lauroyl macrogol-6-glycerides (Labrafil® M2130CS) in an amount of 3% w/w, said composition does not comprise econazole nitrate.

2. The pharmaceutical composition according to claim 1, wherein said composition comprises benzydamine in an amount higher than 0.01% w/w with respect to the total weight of the pharmaceutical composition.

3. The pharmaceutical composition according to claim 1, wherein said composition comprises at least one ester of glycerin with a C₈-C₁₀ saturated fatty acid in an amount lower than 10% w/w with respect to the total weight of the pharmaceutical composition.

4. The pharmaceutical composition according to claim 1, wherein said composition comprises at least one ester of glycerin with a C₈-C₁₀ saturated fatty acid in an amount higher than 0.5% w/w with respect to the total weight of the pharmaceutical composition.

5. The pharmaceutical composition according to claim 1, wherein said composition comprises at least one ester of polyethylene glycol glycerides with a C₁₂-C₁₄ saturated fatty acid in an amount lower than 10% w/w with respect to the total weight of the pharmaceutical composition.

6. The pharmaceutical composition according to claim 1, wherein said composition comprises at least one ester of polyethylene glycol glycerides with a C₁₂-C₁₄ saturated fatty acid in an amount higher than 0.5% w/w with respect to the total weight of the pharmaceutical composition.

7. The pharmaceutical composition according to claim 1, wherein said composition comprises at least one ester of polyethylene glycol with a C₁₆-C₁₈ saturated fatty acid in an amount lower than 30% w/w with respect to the total weight of the pharmaceutical composition.

8. The pharmaceutical composition according to claim 1, wherein said composition comprises at least one ester of polyethylene glycol with a C₁₆-C₁₈ saturated fatty acid in an amount higher than 1% w/w with respect to the total weight of the pharmaceutical composition.

9. The pharmaceutical composition according to any one of the preceding claims, wherein said composition comprises an aqueous dispersion of (i) at least one ester of glycerin with a C₈-C₁₀ saturated fatty acid, (ii) at least one ester of polyethylene glycol glycerides with a C₁₂-C₁₄ saturated fatty acid, (iii) at least one ester of polyethylene glycol with a C₁₆-C₁₈ saturated fatty acid, and (iv) benzydamine in an amount equal to or lower than 0.5% w/w with respect to the total weight of the pharmaceutical composition.

10. The pharmaceutical composition according to claim 9, wherein said composition comprises water in an amount higher than 40% w/w with respect to the total weight of the pharmaceutical composition.

11. The pharmaceutical composition according to any one of the preceding claims, wherein said composition is for topical use.

12. The pharmaceutical composition according to claim 11, wherein said composition is prepared in a dosage form selected from the group comprising creams, ointments, lotions, cream-gels, gels, foams, vaginal douches, or solutions for external use.

13. The pharmaceutical composition according to claim 12, wherein said dosage form is cream, cream-gel, or gel.

14. The pharmaceutical composition according to any one of the preceding claims, wherein said composition comprises at least one preservative selected from the group comprising benzoic acid, ascorbic acid, propyl gallate, and mixture thereof.

15. The pharmaceutical composition according to any one of the preceding claims, wherein said composition comprises at least one stabilizers selected from the group comprising ethylenediaminetetraacetic acid (EDTA), N-(hydroxyethyl)-ethylenediaminetriacetic acid (HEDTA), nitrilotriacetic acid (NTA), diethylenetriaminepentaacetic acid (DTPA), and mixture thereof.

## Patentansprüche

1. Pharmazeutische Zusammensetzung, umfassend (i) wenigstens einen Ester von Glycerin mit einer gesättigten C₈-C₁₀-Fettsäure, (ii) wenigstens einen Ester von Polyethylenglycolglyceriden mit einer gesättigten C₁₂-C₁₄-Fettsäure, und (iii) wenigstens einen Ester von Polyethylenglycol mit einer gesättigten C₁₆-C₁₈-Fettsäure und (iv) Benzydamin in einer Menge von 0,5 Gew.-% oder weniger, bezogen auf das Gesamtgewicht der pharmazeutischen Zusammensetzung,
mit der Maßgabe, dass die Zusammensetzung kein Econazolnitrat umfasst, wenn Benzydamin als Benzydamin-Hydrochlorid in einer Menge von 0,12 % Gew./Gew. vorliegt, zusammen mit Caprylic/Capric Triglycerid (Miglyol® 812) in einer Menge von 3 % Gew./Gew., PEG-6- und PEG-32-Palmitostearat/Glycolstearat (Tefose® 63) in einer Menge von 18 % Gew./Gew. und Lauroyl-Macrogol-6-Glyceriden (Labrafil® M2130CS) in einer Menge von 3 % Gew./Gew.

2. Pharmazeutische Zusammensetzung nach Anspruch 1, wobei die Zusammensetzung Benzydamin in einer Menge von mehr als 0,01 Gew.-%, bezogen auf das Gesamtgewicht der pharmazeutischen Zusammensetzung, umfasst.

3. Pharmazeutische Zusammensetzung nach Anspruch 1, wobei die Zusammensetzung wenigstens einen Ester von Glycerin mit einer gesättigten C₈-C₁₀-Fettsäure in einer Menge von weniger als 10 Gew.-%, bezogen auf das Gesamtgewicht der Zusammensetzung, umfasst.

4. Pharmazeutische Zusammensetzung nach Anspruch 1, wobei die Zusammensetzung wenigsten einen Ester von Glycerin mit einer gesättigten C₈-C₁₀-Fettsäure in einer Menge von mehr als 0,5 Gew.-%, bezogen auf das Gesamtgewicht der Zusammensetzung, umfasst.

5. Pharmazeutische Zusammensetzung nach Anspruch 1, wobei die Zusammensetzung wenigstens einen Ester von Polyethylenglycolglyceriden mit einer gesättigten C₁₂-C₁₄-Fettsäure in einer Menge von weniger als 10 Gew.-%, bezogen auf das Gesamtgewicht der Zusammensetzung, umfasst.

6. Pharmazeutische Zusammensetzung nach Anspruch 1, wobei die Zusammensetzung wenigsten einen Ester von Polyethylenglycolglyceriden mit einer gesättigten C₁₂-C₁₄-Fettsäure in einer Menge von mehr als 0,5 Gew.-%, bezogen auf das Gesamtgewicht der Zusammensetzung, umfasst.

7. Pharmazeutische Zusammensetzung nach Anspruch 1, wobei die Zusammensetzung wenigstens einen Ester von Polyethylenglycol mit einer gesättigten C₁₆-C₁₈-Fettsäure in einer Menge von weniger als 30 Gew.-%, bezogen auf das Gesamtgewicht der Zusammensetzung, umfasst.

8. Pharmazeutischen Zusammensetzung nach Anspruch 1, wobei die Zusammensetzung wenigstens einen Ester von Polyethylenglycol mit einer gesättigten C₁₆-C₁₈-Fettsäure in einer Menge von mehr als 1 Gew.-%, bezogen auf das Gesamtgewicht der Zusammensetzung, umfasst.

9. Pharmazeutische Zusammensetzung nach einem der vorhergehenden Ansprüche, wobei die Zusammensetzung eine wässrige Dispersion von (i) wenigstens einem Ester von Glycerin mit einer gesättigten C₈-C₁₀-Fettsäure, (ii) wenigstens einem Ester von Polyethylenglycolglyceriden mit einer gesättigten C₁₂-C₁₄-Fettsäure, (iii) wenigstens einem Ester von Polyethylenglycol mit einer gesättigten C₁₆-C₁₈-Fettsäure und (iv) Benzydamin in einer Menge von 0,5 Gew.-% oder weniger, bezogen auf das Gesamtgewicht der pharmazeutischen Zusammensetzung, umfasst.

10. Pharmazeutische Zusammensetzung nach Anspruch 9, wobei die Zusammensetzung Wasser in einer Menge von mehr als 40 Gew.-%, bezogen auf das Gesamtgewicht der pharmazeutischen Zusammensetzung, umfasst.

11. Pharmazeutische Zusammensetzung nach einem der vorhergehenden Ansprüche, wobei die Zusammensetzung zur topischen Anwendung bestimmt ist.

12. Pharmazeutische Zusammensetzung nach Anspruch 11, wobei die Zusammensetzung in einer Darreichungsform hergestellt ist, die ausgewählt ist aus der Gruppe, die Cremes, Salben, Lotionen, Creme-Gele, Gele, Schäume, Vaginalspülungen oder Lösungen zur äußerlichen Anwendung umfasst.

13. Pharmazeutische Zusammensetzung nach Anspruch 12, wobei die Darreichungsform eine Creme, ein Creme-Gel oder ein Gel ist.

14. Pharmazeutische Zusammensetzung nach einem der vorhergehenden Ansprüche, wobei die Zusammensetzung wenigstens ein Konservierungsmittel umfasst, das aus der Gruppe ausgewählt ist, die Benzoesäure, Ascorbinsäure, Propylgallat und Mischungen davon umfasst.

15. Pharmazeutische Zusammensetzung nach einem der vorhergehenden Ansprüche, wobei die Zusammensetzung wenigstens einen Stabilisator umfasst, der aus der Gruppe ausgewählt ist, die Ethylendiamintetraessigsäure (EDTA), N-(Hydroxyethyl)-ethylendiamintriessigsäure (HEDTA), Nitrilotriessigsäure (NTA), Diethylentriaminpentaessigsäure (DTPA) und Mischungen davon umfasst.

## Revendications

1. Composition pharmaceutique comprenant (i) au moins un ester de glycérine avec un acide gras saturé en C₈ à C₁₀, (ii) au moins un ester de glycérides de polyéthylène glycol avec un acide gras saturé en C₁₂ à C₁₄, et (iii) au moins un ester de polyéthylène glycol avec un acide gras saturé en C₁₆ à C₁₈, et (iv) de la benzydamine en une quantité égale ou inférieure à 0,5 % p/p par rapport au poids total de la composition pharmaceutique,
à condition que, lorsque la benzydamine est présente sous forme de chlorhydrate de benzydamine en une quantité de 0,12% p/p, conjointement avec du triglycéride caprylique/caprique (Miglyol® 812) en une quantité de 3 % p/p, du palmitostéarate de PEG-6 et PEG-32/stéarate de glycol (Tefose® 63) en une quantité de 18% p/p, et des lauroylmacrogol-6-glycérides (Labrafil® M2130CS) en une quantité de 3 % p/p, ladite composition ne comprend pas de nitrate d'éconazole.

2. Composition pharmaceutique selon la revendication 1, dans laquelle ladite composition comprend de la benzydamine en une quantité supérieure à 0,01 % p/p par rapport au poids total de la composition pharmaceutique.

3. Composition pharmaceutique selon la revendication 1, dans laquelle ladite composition comprend au moins un ester de glycérine avec un acide gras saturé en C₈ à C₁₀ en une quantité inférieure à 10 % p/p par rapport au poids total de la composition pharmaceutique.

4. Composition pharmaceutique selon la revendication 1, dans laquelle ladite composition comprend au moins un ester de glycérine avec un acide gras saturé en C₈ à C₁₀ en une quantité supérieure à 0,5 % p/p par rapport au poids total de la composition pharmaceutique.

5. Composition pharmaceutique selon la revendication 1, dans laquelle ladite composition comprend au moins un ester de glycérides de polyéthylène glycol avec un acide gras saturé en C₁₂ à C₁₄ en une quantité inférieure à 10 % p/p par rapport au poids total de la composition pharmaceutique.

6. Composition pharmaceutique selon la revendication 1, dans laquelle ladite composition comprend au moins un ester de glycérides de polyéthylène glycol avec un acide gras saturé en C₁₂ à C₁₄ en une quantité supérieure à 0,5 % p/p par rapport au poids total de la composition pharmaceutique.

7. Composition pharmaceutique selon la revendication 1, dans laquelle ladite composition comprend au moins un ester de polyéthylène glycol avec un acide gras saturé en C₁₆ à C₁₈ en une quantité inférieure à 30 % p/p par rapport au poids total de la composition pharmaceutique.

8. Composition pharmaceutique selon la revendication 1, dans laquelle ladite composition comprend au moins un ester de polyéthylène glycol avec un acide gras saturé en C₁₆ à C₁₈ en une quantité supérieure à 1 % p/p par rapport au poids total de la composition pharmaceutique.

9. Composition pharmaceutique selon l'une quelconque des revendications précédentes, dans laquelle ladite composition comprend une dispersion aqueuse de (i) au moins un ester de glycérine avec un acide gras saturé en C₈ à C₁₀, (ii) au moins un ester de glycérides de polyéthylène glycol avec un acide gras saturé en C₁₂ à C₁₄, et (iii) au moins un ester de polyéthylène glycol avec un acide gras saturé en C₁₆ à C₁₈, et (iv) de la benzydamine en une quantité égale ou inférieure à 0,5 % p/p par rapport au poids total de la composition pharmaceutique.

10. Composition pharmaceutique selon la revendication 9, dans laquelle ladite composition comprend de l'eau en une quantité supérieure à 40 % p/p par rapport au poids total de la composition pharmaceutique.

11. Composition pharmaceutique selon l'une quelconque des revendications précédentes, dans laquelle ladite composition est pour un usage topique.

12. Composition pharmaceutique selon la revendication 11, dans laquelle ladite composition est préparée sous une forme posologique choisie dans le groupe comprenant les crèmes, les pommades, les lotions, les crèmes-gels, les gels, les mousses, les douches vaginales ou les solutions à usage externe.

13. Composition pharmaceutique selon la revendication 12, dans laquelle ladite forme posologique est une crème, une crème-gel ou un gel.

14. Composition pharmaceutique selon l'une quelconque des revendications précédentes, dans laquelle ladite composition comprend au moins un conservateur choisi dans le groupe comprenant l'acide benzoïque, l'acide ascorbique, le gallate de propyle et un mélange de ceux-ci.

15. Composition pharmaceutique selon l'une quelconque des revendications précédentes, dans laquelle ladite composition comprend au moins un stabilisant choisi dans le groupe comprenant l'acide éthylènediaminetétraacétique (EDTA), l'acide N-(hydroxyéthyl)-éthylènediaminetriacétique (HEDTA), l'acide nitrilotriacétique (NTA), l'acide diéthylènetriaminepentaacétique (DTPA) et un mélange de ceux-ci.
